# EUROPÄISCHE PATENTSCHRIFT

(11) **EP 3 072 540 B1**
(45) Veröffentlichungstag und Bekanntmachung des Hinweises auf die Patenterteilung: **02.08.2017**
(21) Anmeldenummer: 16158660.7
(22) Anmeldetag: 04.03.2016
(51) Int. Cl.: A61L 31/02, A61L 31/08, A61L 31/14, A61L 31/16

(54) **KNOCHENSCHRAUBE, ORTHOPÄDISCHES IMPLANTAT UND VERFAHREN ZUR HERSTELLUNG DIESER**
BONE SCREW, ORTHOPEDIC IMPLANT AND METHOD FOR PRODUCING SAME
VIS A OS, IMPLANT ORTHOPEDIQUE ET LEUR PROCEDE DE FABRICATION

(30) Priorität: 24.03.2015 US 201562137239 P
(43) Veröffentlichungstag der Anmeldung: 28.09.2016
(73) Patentinhaber: Biotronik AG, 8180 Bülach (CH)
(72) Erfinder: Bayer, Ullrich, 18209 Bad Doberan (DE); Lootz, Daniel, 18119 Rostock (DE)
(74) Vertreter: Galander, Marcus

(56) Entgegenhaltungen:
- US-A1- 2014 243 911

## Beschreibung

Die Erfindung betrifft eine Knochenschraube mit selbstschneidendem Gewinde sowie ein orthopädisches Implantat sowie schließlich ein Verfahren zur Herstellung solcher Knochenschrauben bzw. Implantate.
Knochenschrauben mit selbstschneidendem Gewinde sind seit Jahrzehnten bekannt und stellen wesentliche Befestigungselemente in der Prothetik dar. Sie sind während der bereits langen Zeitdauer ihres klinischen Einsatzes Gegenstand vielfältiger Verbesserungen sowohl hinsichtlich des Materials als auch hinsichtlich der geometrischen Konfiguration gewesen. Den breitesten Einsatz haben in der Vergangenheit Edelstahl- und Titanschrauben gefunden. Es hat jedoch immer wieder Bestrebungen gegeben, resorbierbare Knochenschrauben und ähnliche orthopädische Implantate mit vergleichbaren Leistungsparametern bereitzustellen. In diesem Zusammenhang sind Knochenschrauben aus Magnesiumlegierungen bekannt geworden; siehe etwa US 2011/0313527 A1, und auch Schrauben auf Mg-Basis mit speziellen Beschichtungen, wie in US 2012/0150295 A1 beschrieben. Die Druckschrift US 2014/243911 A offenbart eine Knochenschraube aus einer Magnesiumlegierung, die zur Erhöhung der Bruchfestigkeit einen verdickten Schraubenkopf aufweist.

Magnesium besitzt im Vergleich zu Titan und Edelstählen geringere Festigkeiten. Dies macht den Einsatz dieser Werkstoffgruppe für selbstschneidende resorbierbare Knochenschrauben die mit einem hohen Drehmoment in das Knochengewebe eingebracht werden müssen problematisch. Besonders bei kleinformatigen und/oder kanülierten Schrauben besteht die Gefahr, dass das anzulegende Drehmoment die Torsionsfestigkeit des Werkstoffs übersteigt und somit die Schraube überdreht wird und bricht.
Der Erfindung liegt daher die Aufgabe zugrunde, eine Knochenschraube bzw. ein entsprechendes orthopädisches Implantat mit verringerter Bruchgefahr und der sich hieraus ergebenden Option einer weiteren Miniaturisierung bereitzustellen. Des Weiteren soll ein Verfahren zur Herstellung derartiger Schrauben bzw. Implantate bereitgestellt werden.

Diese Aufgabe wird in ihrem Vorrichtungsaspekt durch eine Knochenschraube mit den Merkmalen des Anspruchs 1 bzw. ein orthopädisches Implantat mit den Merkmalen des Anspruchs 9 und in ihrem Verfahrensaspekt durch ein Verfahren mit den Merkmalen des Anspruchs 10 gelöst. Zweckmäßige Fortbildungen des Erfindungsgedankens sind Gegenstand der abhängigen Ansprüche.

Die Erfindung schließt die Überlegung ein, durch eine Oberflächenfunktionalisierung des selbstschneidenden Gewindeprofils die Reibung beim Eindrehen der Schrauben zu minimieren. Dadurch wird das notwendige Dreh- bzw. Torsionsmoment verringert und die Gefahr des Schraubenbruchs wesentlich reduziert. Dieses oberflächenfunktionalisierte, selbstschneidende und selbstschmierende Gewindeprofil soll gemäß einer weiteren Überlegung der Erfinder zudem noch so gestaltet sein, dass während und unmittelbar nach dem Schneidvorgang die Knochenheilung beschleunigende Substanzen (wie z. B. eine Proliferation humaner Knochenzellen begünstigende bmp's) freigesetzt werden. Das Gewinde weist dadurch eine multifunktionale Oberfläche auf. Gemäß einem weiteren Aspekt der Erfindung wird zusätzlich oder alternativ zur erwähnten Minimierung der Reibung beim Eindrehen der Schraube eine Erhöhung der Selbstschneidwirkung durch einen entsprechend lokalisierten Zusatzstoff vorgeschlagen. Dieser liegt erfindungsgemäß in Form von Mikro-Schleifkörpern in der bioaktiven Oberflächenbeschichtung vor.

Mit anderen Worten: Die Schneidhaltigkeit soll einerseits durch ebenfalls degradierbare Verbindungen erreicht werden (stofflicher Aspekt), anderseits soll durch die mit dieser zum Einsatz kommenden Schleifkörper die Oberflächenrauheit gezielt erhöht werden (oberflächentopographischer Aspekt). Durch die dadurch entstehende Vergrößerung der realen Oberfläche werden Mikrokavitäten erzeugt, die eine bessere Verankerung von Mikropartikeln ermöglichen. Diese bestehen insbesondere aus polymeren Hohlkugeln, die mit das Knochenwachstum fördernden Substanzen gefüllt sind.

Beim Schneiden bzw. Eindrehen der Schraube in die Knochenspongiosa entstehen lokale Reibmomente an den Gewindespitzen und den Gewindeflanken. Diese führen einerseits zu einer Fragmentierung des harten und spröden Schneidstoffs, andererseits zu kurzzeitig wirkenden, lokal begrenzten Temperaturerhöhungen. Beide Effekte führen zu einer gewollten Zerstörung des polymeren Hüllenmaterials in deren Ergebnis die das Knochenwachstum stimulierenden Wirkstoffe in flüssiger oder pastöser Form freigesetzt werden.

Durch die verbesserte Schneidwirkung wird die Reibung während des Einschraubvorgangs minimiert. Die damit einhergehende geringere Torsionsbelastung der Schraube ermöglicht eine Querschnitts- und damit eine Massereduzierung. Dies leistet einerseits einen wesentlichen Beitrag zur Minimierung der Degradationszeit und reduziert andererseits die Menge an frei werdendem Wasserstoff. Damit wird schlussendlich eine größere Körperverträglichkeit und eine Reduzierung der Rehabilitationszeit der Patienten erreicht. Außerdem kann eine weitere Miniaturisierung der Schrauben erreicht werden, und es können somit neue Einsatzgebiete resorbierbarer Schrauben im Schädel- und Kleingliedmaßenbereich erschlossen werden.

In einer Ausführung der Erfindung hat die Knochenschraube einen kanülierten Grundkörper, insbesondere mit einem Außendurchmesser im Bereich zwischen 1,5 und 5 mm, spezieller zwischen 2,5 und 3,5 mm, und einem Innendurchmesser im Bereich zwischen 0,5 und 2,5 mm, spezieller zwischen 0,8 und 1,3 mm. Auch hierdurch wird eine Massenreduzierung der Schraube erreicht. Diese Reduktion der Menge an Magnesium führt wiederum zu einer verringerten Degradationszeit und gleichzeitig zu einer Verringerung der Menge an Wasserstoff, der bei der Degradation freigesetzt wird. Dies wiederum verringert die Gefahr der taschenförmigen Einlagerung von Wasserstoff, der vom Körper nicht schnell genug resorbiert werden kann.

In einer weiteren Ausführung sind die Mikro-Schleifkörper der Oberflächenbeschichtung durch Druck und Reibungswärme beim Eindrehen der Knochenschraube in die Knochensubstanz zerstörbar, insbesondere in kleinere Partikel umwandelbar, die eine Vergrößerung der effektiven Schraubenoberfläche bewirken. Dies erhöht die wirksame Schneidkraft der Schraube gegenüber dem Knochengewebe und somit letztlich die Selbstschneidwirkung.

Auch hieraus ergibt sich wiederum die Möglichkeit einer Reduzierung der Schraubendimensionen, mit den oben erwähnten Vorteilen. Die sich aus den größeren Schleifkörpern bildenden kleinen Partikel bewirken zudem ein schnelleres Einwachsen der Schraube in die Knochenmatrix.

In einer weiteren Ausführung der Erfindung weisen die Mikro-Schleifkörper kristallines Hydroxylapatit auf und sind insbesondere nadelförmig ausgebildet. Die typische Form dieses kommerziell verfügbaren Schleifmittels eignet sich, insbesondere bei entsprechend orientierter Einbindung der Schleifkörper in die Oberflächenbeschichtung, besonders zur Steigerung der Schneidfähigkeit und Schneidhaltigkeit der vorgeschlagenen Knochenschraube.

In einer weiteren Ausführung der Erfindung enthalten die Mikrokapseln knochenmorphogenetische Proteine. Die Wechselwirkung der bioaktiven Oberfläche mit der Knochensubstanz startet hierbei unverzüglich mit dem Eindrehen der Schraube in die Knochenspongiosa. Die beim Eindrehvorgang entstehende Reibungswärme und die Fragmentierungsprozesse des Schneidenmaterials aktivieren die im Gewindeprofil befindlichen, mit biodegradierbaren Polymeren umhüllten knochenmorphogenetischen Proteine. Diese kommen dann sofort in den Kontakt mit der Spongiosa einerseits und dem Schraubenmaterial andererseits.

In weiteren Ausführungen ist vorgesehen, dass die Mikrokapseln eine Biopolymer-Hülle haben, die insbesondere ein Polylactid und spezieller ein PLA-Blend, aufweisen. Hierbei handelt es sich um leicht und kostengünstig verfügbare Hüllenmaterialien, die gut für ein biodegradierbares Produkt geeignet sind und deren Einsatz die Kosten der zusätzlichen Beschichtung niedrig zu halten hilft.

In weiteren Ausführungen der Erfindung weisen die Mikrokapseln und/oder die Mikro-Schleifkörper, letztere in ihrer größten Erstreckung, eine Größe im Bereich zwischen 0,2 und 20 µm, insbesondere zwischen 1 und 10 µm, auf. Mit diesen Dimensionen lassen sich hoch wirksame funktionale Beschichtungen von Schrauben aller praktisch bedeutenden Dimensionen mit etablierten Beschichtungstechniken realisieren, und die Beschichtungen sind einerseits hinreichend wirksam und andererseits ausreichend standfest.

In einer weiteren Ausführung der Erfindung weist das selbstschneidende Gewinde ein Sägefeingewindeprofil auf Grundsätzlich sind auch andere Profilquerschnitte, insbesondere solche mit im Querschnitt gekrümmten Gewindeflanken, möglich. Deren Herstellung ist jedoch möglicherweise aufwändiger als die eines herkömmlichen Sägefeingewindeprofils mit geradlinig verlaufenden Gewindeflanken und daher aus heutiger Sicht nicht bevorzugt.

Gemäß dem vorgeschlagenen Verfahren umfasst die Herstellung einer Knochenschraube oder eines orthopädischen Implantats der oben erläuterten Art die Schritte:
- Bereitstellen eines Rohres oder eines einen Rohrabschnitt aufweisenden Grundkörpers aus einer Magnesiumlegierung,
- Einformen des selbstschneidenden Gewindes in die Außenwandung des Rohres oder Rohrabschnitts zur Herstellung der Knochenschraube oder des Knochenschraubenabschnitts des orthopädischen Implantats,
- Ausbilden einer Beschichtungs-Basis auf mindestens einem Abschnitt der Oberfläche des Gewindes, und
- Auf-/Einbringen der Mikrokapseln und/oder der Mikro-Schleifkörper auf und/oder in die Beschichtungs-Basis.
Grundsätzlich kann zwar der Grundkörper einer erfindungsgemäßen Knochenschraube oder eines entsprechenden orthopädischen Implantats auch in einem Gieß- oder Druckgußverfahren hergestellt werden, dies hat jedoch normalerweise höhere Kosten zur Folge.

In Ausführungen des vorgeschlagenen Verfahrens weist das Einformen des selbstschneidenden Gewindes ein rotierendes Strangpressen mit nachfolgendem Fräsen oder Schleifen oder ein Fräsen oder ein Gewindeschneiden auf. Die konkret bevorzugte Technik der Gewindeherstellung kann sich nach einschlägigen Erfahrungen und verfügbaren Maschinen des Herstellers richten.

In einer weiteren Ausführung des Verfahrens weist das Ausbilden der Beschichtungs-Basis ein Eintauchen in eine Beschichtungsflüssigkeit, insbesondere eine ein Polylactid und spezieller ein PLA-Blend enthaltende Flüssigkeit, und nachfolgendes Trocknen bei erhöhter Temperatur auf. Grundsätzlich kommen aber auch Sprühbeschichtungs- und andere bekannte Flüssigkeits-Beschichtungsverfahren in Betracht. Analog weist bevorzugt auch das Auf-/Einbringen der Mikrokapseln ein Eintauchen in eine Mikrokapseln enthaltende Flüssigkeit mit nachfolgendem Trocknen bei erhöhter Temperatur auf, und schließlich kann auch das Auf-/Einbringen der Mikro-Schleifkörper ein Eintauchen in eine Mikro-Schleifkörper enthaltende Flüssigkeit mit nachfolgendem Trocknen bei erhöhter Temperatur aufweisen.

Bei allen vorgenannten Verfahrensführungen kann das Auf-/Einbringen der Mikrokapseln und/oder Mikro-Schleifkörper ein vorangehendes Anlösen der Oberfläche der Beschichtungs-Basis zur Schaffung einer mikrorauhen Haftfläche aufweisen. Der mit dem zusätzlichen Schritt verbundene erhöhte Aufwand kann durch die erzielbare Erhöhung der Standfestigkeit der Beschichtung völlig gerechtfertigt sein.

Vorteile und Zweckmäßigkeiten der Erfindung ergeben sich im Übrigen aus der nachfolgenden Beschreibung von Ausführungsbeispielen anhand der Figuren. Von diesen zeigen:
- Fig. 1A bis 1C: Darstellungen einer erfindungsgemäßen Knochenschraube bzw. eines zugehörigen Kreuzstiftes zum Einschrauben derselben,
- Fig. 2: eine Detailansicht aus Fig. 1A und
- Fig. 3A und 3B: Detailansichten aus Fig. 2 zu Ausführungsformen der Erfindung.

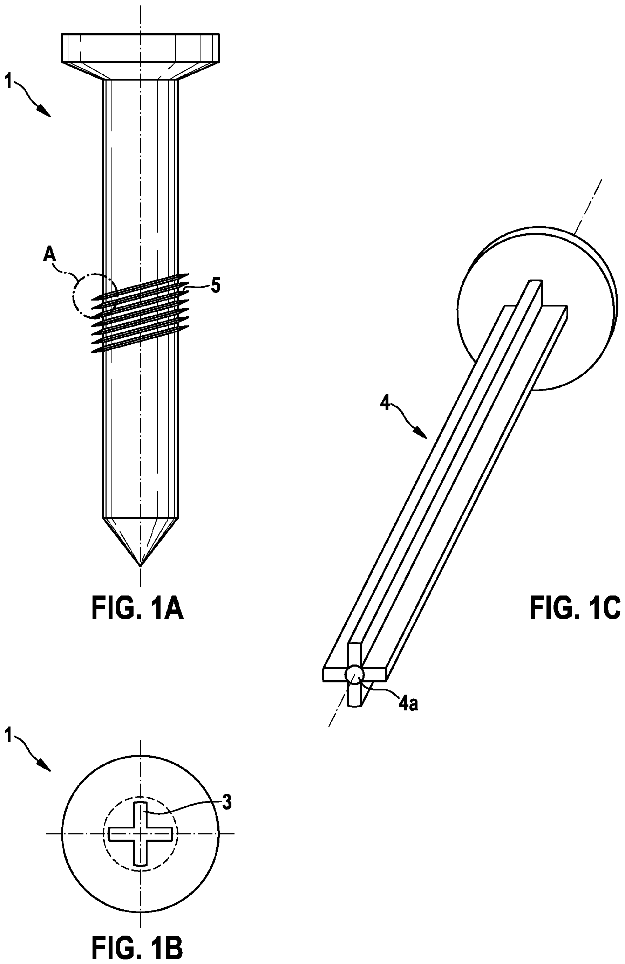

Fig. 1A zeigt in einer skizzenartigen Darstellung (Seitenansicht) eine Knochenschraube 1 aus einer Mg-Legierung, die innen hohl bzw. kanüliert gefertigt ist, und zwar - wie in der Draufsicht auf den Schraubenkopf gemäß Fig. 1B zu erkennen ist - mit einem kreuzschlitzförmigen Hohlprofil 3. Fig. 1C zeigt in einer schematischen perspektivischen Darstellung einen zur Knochenschraube 1 nach Fig. 1A und 1B passenden Kreuzstift 4, in dessen Innerem eine Längsbohrung 4a zum Einführen eines Führungsdrahtes vorgesehen ist.

Die oben kurz beschriebene Konfiguration hat zum einen den Vorteil, dass die Knochenschraube mit geringerem Materialeinsatz und somit einer geringeren Menge von im Körper korrodierendem und abzubauendem Material zu fertigen ist. Zum anderen ermöglicht es das Vorsehen des kreuzschlitzförmigen Hohlprofils in Verbindung mit der Benutzung des bevorzugt aus einer Stahl- oder Titanlegierung gefertigten Kreuzstiftes, beim Eindrehen der Schraube höhere Drehmomente zu übertragen als bei einer Knochenschraube, die massiv aus einer Mg-Legierung gefertigt ist.

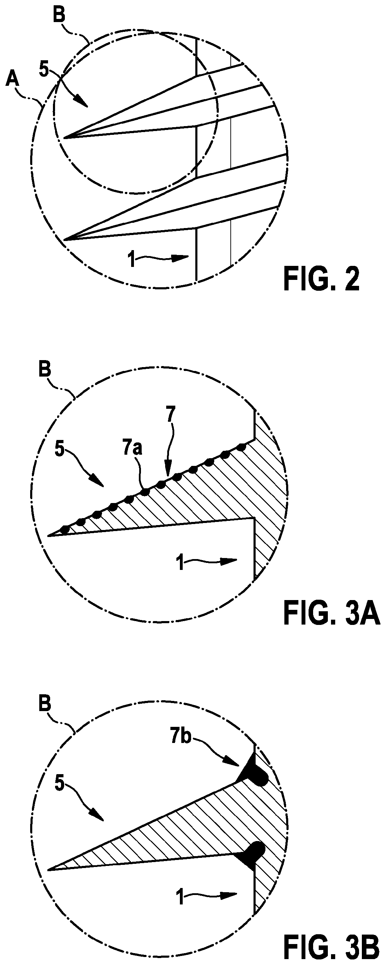

Fig. 2 zeigt in einer schematischen Seitenansicht das Detail A des Schraubengewindes 5 der Knochenschraube 1. Es ist zu erkennen, dass es sich hierbei um ein sehr spitzwinkliges, "scharfes" Sägefeingewindeprofil handelt. Bei der gezeigten Ausführung beträgt der Winkel zwischen den Gewindeflanken ca. 20°, er kann aber in Abhängigkeit vom konkreten Material und Einsatzzweck der Schraube auch einen anderen Wert, insbesondere zwischen 15° und 30°, haben.

Fig. 3A zeigt in einer weiter vergrößerten Ansicht das Detail B aus Fig. 2 in einer Schnittansicht, und zwar speziell eine in Hohlräumen der Gewindeflanke des Schraubengewindes 5 "verankerte" Oberflächenbeschichtung 7 mit eingelagerten Mikro-Schleifkörpern 7a. In der weiteren Detailansicht der Fig. 3B ist (unter Fortlassung der Oberflächenbeschichtung 7) speziell nur gezeigt, dass in einer Ausführung der Erfindung an den Übergängen des Grundkörpers der Knochenschraube 1 zum Gewindeprofil 5 Mikro-Reservoire (Mikrokapseln) 7b eingelagert sind, die mit einem an das Schraubenmaterial einerseits und die Knochensubstanz andererseits angepassten Schmierstoff und gegebenenfalls auch einem medizinischen Wirkstoff befüllt sind. Diese Mikrokapseln 7b können mit den in Fig. 3A gezeigten Mikro-Schleifkörpern 7a kombiniert sein.

Die Mikrokapseln 7b adhärieren sowohl an der Kavitäten aufweisenden, mit mikrokristallinem Hydroxylapatit bedeckten Oberfläche des Gewindeprofils 5 als auch stoff- und formschlüssig in der dünnen, aus einem biodegradierbaren Polymer bestehenden Oberflächenschicht 7, die als Wirtsmatrix für die Mikro-Schleifkörper 7a, etwa aus Hydroxylapatit, fungiert. Es entsteht ein Oberflächenverbund aus hartem und schneidfähigen Hydroxylapatitkristallen und dazwischen eingelagerten Mikrokapseln, die wiederum aus einem Hüllenmaterial aus biodegradabierbaren Polymer und darin enthaltenden Wirkstoffen für die Osteosynthese bestehen.

Die Wirkstoffe enthaltenden Mikrokapseln werden beim Schneidvorgang durch die eintretende Fragmentierung der harten, aber auch spröden Hydroxylapatitkörper mechanisch und lokal auch durch thermische Effekte (Mikroreibungswärme) zerstört. Die heraustretende hochviskose wirkstoffbeladene Trägerflüssigkeit ruft einen Selbstschmiereffekt hervor, infolge dessen das aufzubringende Eindrehmoment der Schraube noch weiter reduziert wird. Durch die nun vorliegende wesentliche Reduzierung des Eindrehmoments ergibt sich eine geringere Torsionsbelastung der Schraube. Diese führt zu der konstruktiven Möglichkeit, die Wandstärke der kanülierten Schraube und damit deren Masse im Bereich zwischen 20 und 50 % zu reduzieren. Dadurch wird einerseits eine Festigkeitsreserve geschaffen, die eine weitere Miniaturisierung von Knochenschrauben ermöglicht, ohne die Gefahr des Abdrehens der Schraube durch zu hohe Torsionsbelastung zu erhöhen. Andererseits wird die Degradationsdauer verkürzt und die Menge an freigegebenen Wasserstoff reduziert.

Hydroxylapatite gehören zur Gruppe der Calciumphosphate, die als Knochenersatzwerkstoffe keine körpereigenen Abwehrreaktionen hervorrufen. Hydroxylapatite sind jedoch spröde. Dies drückt sich durch geringe Bruchzähigkeitskennwerte (K_{IC} = 1MPam^{1/2}) aus. Aus diesem Grunde finden Hydroxylapatite in der Regel keine Anwendung für lasttragende orthopädische Implantate, da diese zur Rissbildung neigen und dadurch mechanisch nicht mehr belastbar wären.

Die erfindungsgemäße Lösung nutzt in einer Ausführungsform diesen vermeintlichen Nachteil ganz gezielt aus. Die in der Magnesiummatrix der Schraube eingelagerten Hydroxylapatitkristalle (Partikelgrößenbereich z.B. 1-5 µm) werden beim Einschraubvorgang komplexen mechanischen Beanspruchungen ausgesetzt. Diese sind gekennzeichnet durch Zug- und Druckspannungen, die teilweise durch Torsionsspannungen überlagert werden. Diese komplexen Spannungen sind bei weitem größer als die Festigkeit der Hydroxylapatitkristalle, und es kommt zur Fragmentierung jener Kristalle. Es entstehen mikrokristalline Partikel (0,1 -1 µm) mit viel größeren realen Oberflächen, die zu einem Teil weiterhin in der Magnesiumoberfläche der Schraube verankert bleiben, zum anderen Teil jedoch von der Oberfläche weg und - unterstützt durch die in den Mikrokapseln enthaltende Flüssigkeit - in die umgebende Knochenspongiosa transportiert werden. Diese Kette von biologisch äußerst erwünschten Effekten ist mit keiner anderen Lösung erzielbar und sei nochmals im Folgenden dargestellt:
- Das Eindrehen der Schraube in die Knochenspongiosa wird durch die Schneidwirkung der noch großen Hydroxylapatitkristalle erleichtert; ein geringeres Eindrehmoment der Schraube schützt vor Überdrehung dieser.
- Die gleichzeitig einsetzende Fragmentierung der Hydroxylapatitkristalle führt zu kleineren Partikeln die eine insgesamt größere reale Oberfläche aufweisen. Das Calciumphosphat der Hydroxylapatitkristalle steht nunmehr rascher zur Verfügung.
- Dies führt in Verbindung mit der nunmehr freigesetzten wirkstoffbeladenen Trägerflüssigkeit und den Fragmenten des degradierbaren Kapselmaterials zu einem höheren Grad an Bioaktivierung durch Vorortsynthese eines bioaktiven Composites.

Dieses Composit stimuliert eine viel früher einsetzende Bildung von Osteoblasten, mit dem Ergebnis einer rascheren Bildung von neuer Knochensubstanz.

Eine Modifikation der o.a. Ausführung besteht darin, den mikrokristallinen Partikeln (1-5 µm) amorphes Titanoxidpulver (TiO2) in einer Konzentration von z.B. 5-10 Masse-% beizumischen. Dessen Partikelgröße kann insbesondere im Bereich zwischen 50 und 100 nm liegen. Damit wird oben genannter Effekt erreicht, dass es beim Eindrehen der Knochenschraube in die Spongiosa zu einer Schneidwirkung des Hydroxylapatits bei einer gleichzeitig einsetzenden Fragmentierung kommt. Somit tritt eine Kombination folgender, wünschenswerter und erfindungsgemäßer Effekte auf:
- Das Torsionsmoment, das zum Eindrehen der resorbierbaren Schraube in den Knochen benötigt wird, wird nicht wesentlich erhöht, und die Gefahr des "Überdrehens" der Schraube vor Erreichung der geforderten Einschraubtiefe wird ausgeschlossen.
- Die Schneidwirkung der in bzw. an der Gewindeoberfläche eingelagerten Hydroxylapatitkristalle bleibt trotzdem erhalten. Durch diesen Schneidprozess werden aktive Spongiosaoberflächen erzeugt. Diese zeigen erhöhte biologische Wechselwirkungen zum Hydroxylapatit, die letztendlich eine Beschleunigung des Einheilprozesses der Schraubenoberfläche bewirken.
- Durch die Zugabe des bioinerten nanoskaligen Titanoxidpulvers erfolgt eine Fragmentierung der Hydroxylapatitkristalle in der Art, dass eine Oberflächenvergrößerung erfolgt, die in Verbindung mit dem gleichzeitig freigesetzten wirkstoffbeladenen Kapselmaterial den Effekt einer beschleunigten Inkorporation noch mehr begünstigt. Das Titanoxid verbleibt ohne weitere chemische Wechselwirkung (und damit auch ohne negative Einflüsse auf die Degradation der Schraube) in den Zwischenräumen der Spongiosa.

Eine beispielhafte Ausführung der Erfindung unter material- und verfahrenstechnischen Gesichtspunkten sieht wie folgt aus:
1. Es wird ein extrudiertes Rohr aus der biodegradierbaren Mg-Legierung WE 43 mit: AD= 2,5 mm; ID= 0,8mm; daraus resultierende Wanddicke = 0,85 mm bereitgestellt.
2. Es wird ein Schrauben-Grundkörper in einer Länge von 15 mm bis 40 mm abgetrennt.
3. Es erfolgt ein mechanisches Schneiden (mit Schneideisen) eines selbstschneidenden Sägenfeingewindeprofils (Gewindetiefen von 0,5 bis 0,7mm) mit einer Gewindesteigung zwischen 0,5 und 1,5 mm/ Umdrehung.
4. Es erfolgt eine Reinigung in Isopropanol (Verweilzeit 2 min).
5. Es erfolgt ein Tauchen der Schraube (dip coating) in eine Flüssigkeit aus PLA oder PLA Blends, die Hydroxylapatitpartikel (Partikelgröße zwischen 1 und 10 µm) enthält, bei mindestens 150 °C.
6. Es erfolgt ein Trocknen in einem Umluftofen bei Temperaturen zwischen 60 und 80°C.
7. Es erfolgt ein kurzes Eintauchen (<10 s) der beschichteten Schraube in Chloroform; dadurch wird ein Teil der Oberfläche angelöst. Es entsteht eine selbstklebende, mit Mikrokavitäten versehene mikrorauhe Oberfläche.
8. Danach erfolgt ein sofortiges Herausnehmen und Eintauchen (10-30 s) in eine wässrige Mikrokapseln enthaltende Flüssigkeit. Diese Mikrokapseln bestehen aus einer Hülle aus einem PLA oder PLA Blend. Der Durchmesser dieser Mikrokapseln beträgt zwischen 1 und 10 µm. Diese Mikrokapseln enthalten in ihrem Inneren knochenmorphogenetische Proteine.
9. Es erfolgt ein Herausnehmen und Trocknen in einem Umluftofen bei Temperaturen zwischen 30 und 40°C. Ein Teil der Mikrokapseln hat sich in die Mikrokavitäten der PLA/Hydroxylapatit enthaltenden Oberfläche eingelagert (Formschluss) und/oder klebt an dieser Oberfläche fest (Stoffschluss).

In einer Abwandlung dieser Ausführung kann ein Rohr aus der biodegradierbaren Mg-Legierung MgCa0,8 oder aus der biodegradierbaren Mg-Legierung AZ31 eingesetzt werden. Außen- und Innendurchmesser der Schraube und somit die resultierende Wanddicke können ebenso variiert werden, wie Gewindetiefe und -Steigung und andere Parameter der Schraubenkonfiguration. Auch im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Knochenschraube (1) mit selbstschneidendem Gewinde (5), im Wesentlichen bestehend aus einer Magnesiumlegierung und mindestens in Bereichen des Gewindes versehen mit einer bioaktiven Oberflächenbeschichtung (7), die durch Druck und Reibungswärme beim Eindrehen der Knochenschraube in Knochensubstanz zerstörbare Mikrokapseln (7b) mit einem Schmierstoff (7a) und/oder Mikro-Schleifkörper enthält.

2. Knochenschraube nach Anspruch 1, wobei die Mikro-Schleifkörper (7a) der Oberflächenbeschichtung (7) durch Druck und Reibungswärme beim Eindrehen der Knochenschraube in Knochensubstanz zerstörbar, insbesondere in kleinere Partikel umwandelbar sind, die eine Vergrößerung der effektiven Schraubenoberfläche bewirken.

3. Knochenschraube nach Anspruch 1 oder 2, wobei die Mikro-Schleifkörper (7a) kristallines Hydroxylapatit aufweisen und insbesondere nadelförmig ausgebildet sind.

4. Knochenschraube nach einem der vorangehenden Ansprüche, wobei die Mikrokapseln (7b) knochenmorphogenetische Proteine enthalten.

5. Knochenschraube nach einem der vorangehenden Ansprüche, wobei die Mikrokapseln eine Biopolymer-Hülle aufweisen, die insbesondere ein Polylactid und spezieller ein PLA-Blend, aufweisen.

6. Knochenschraube nach einem der vorangehenden Ansprüche, wobei die Mikrokapseln (7b) und/oder die Mikro-Schleifkörper (7b), letztere in ihrer größten Erstreckung, eine Größe im Bereich zwischen 0,2 und 20 µm, insbesondere zwischen 1 und 10 µm, aufweisen.

7. Knochenschraube nach einem der vorangehenden Ansprüche, wobei das selbstschneidende Gewinde (5) ein Sägefeingewindeprofil aufweist.

8. Knochenschraube nach einem der vorangehenden Ansprüche, welche einen kanülierten Grundkörper, insbesondere mit einem Außendurchmesser im Bereich zwischen 1,5 und 5 mm, spezieller zwischen 2,5 und 3,5 mm, und einem Innendurchmesser im Bereich zwischen 0,5 und 2,5 mm, spezieller zwischen 0,8 und 1,3 mm, aufweist.

9. Orthopädisches Implantat, das eine Knochenschraube (1) nach einem der vorangehenden Ansprüche oder einen entsprechenden angeformten Knochenschraubenabschnitt aufweist.

10. Verfahren zur Herstellung einer Knochenschraube (1) nach einem der Ansprüche 1 bis 8 oder eines orthopädischen Implantats nach Anspruch 9, mit den Schritten:
- Bereitstellen eines Rohres oder eines einen Rohrabschnitt aufweisenden Grundkörpers aus einer Magnesiumlegierung,
- Einformen des selbstschneidenden Gewindes (5) in die Außenwandung des Rohres oder Rohrabschnitts zur Herstellung der Knochenschraube oder des Knochenschraubenabschnitts des orthopädischen Implantats,
- Ausbilden einer Beschichtungs-Basis (7) auf mindestens einem Abschnitt der Oberfläche des Gewindes, und
- Auf-/Einbringen der Mikrokapseln (7b) und/oder der Mikro-Schleifkörper (7a) auf und/oder in die Beschichtungs-Basis.

11. Verfahren nach Anspruch 10, wobei das Einformen des selbstschneidenden Gewindes (5) ein rotierendes Strangpressen mit nachfolgendem Fräsen oder Schleifen oder ein Fräsen oder ein Gewindeschneiden aufweist.

12. Verfahren nach Anspruch 10 oder 11, wobei das Ausbilden der Beschichtungs-Basis (7) ein Eintauchen in eine Beschichtungsflüssigkeit, insbesondere eine ein Polylactid und spezieller ein PLA-Blend enthaltende Flüssigkeit, und nachfolgendes Trocknen bei erhöhter Temperatur aufweist.

13. Verfahren nach einem der Ansprüche 10 bis 12, wobei das Auf-/Einbringen der Mikrokapseln (7b) ein Eintauchen in eine Mikrokapseln enthaltende Flüssigkeit mit nachfolgendem Trocknen bei erhöhter Temperatur aufweist.

14. Verfahren nach einem der Ansprüche 10 bis 13, wobei das Auf-/Einbringen der Mikro-Schleifkörper (7a) ein Eintauchen in eine Mikro-Schleifkörper enthaltende Flüssigkeit mit nachfolgendem Trocknen bei erhöhter Temperatur aufweist.

15. Verfahren nach einem der Ansprüche 10 bis 14, wobei das Auf-/Einbringen der Mikrokapseln (7b) und/oder Mikro-Schleifkörper (7a) ein vorangehendes Anlösen der Oberfläche der Beschichtungs-Basis zur Schaffung einer mikrorauhen Haftfläche aufweist.

## Claims

1. A bone screw (1) with self-tapping thread (5), consisting substantially of a magnesium alloy and provided at least in regions of the thread with a bioactive surface coating (7), which contains microcapsules (7b) with a lubricant (7a) and/or micro-abrasives, which microcapsules can be destroyed by pressure and frictional heat as the bone screw is screwed into bone substance.

2. The bone screw according to claim 1, wherein the micro-abrasives (7a) of the surface coating (7) can be destroyed, in particular can be converted into smaller particles, by pressure and frictional heat as the bone screw is screwed into bone substance, which particles enlarge the effective screw surface.

3. The bone screw according to claim 1 or 2, wherein the micro-abrasives (7a) comprise crystalline hydroxyapatite and in particular are needle-shaped.

4. The bone screw according to any one of the preceding claims, wherein the microcapsules (7b) contain bone morphogenetic proteins.

5. The bone screw according to any one of the preceding claims, wherein the microcapsules comprise a biopolymer shell, which in particular comprises a polylactide and especially a PLA blend.

6. The bone screw according to any one of the preceding claims, wherein the microcapsules (7b) and/or the micro-abrasives (7b), the latter in the greatest extension thereof, have a size in the range between 0.2 and 20 µm, in particular between 1 and 10 µm.

7. The bone screw according to any one of the preceding claims, wherein the self-tapping thread (5) has a saw-like fine-thread profile.

8. The bone screw according to any one of the preceding claims, which has a cannulated main body, in particular with an outer diameter in the range between 1.5 and 5 mm, especially between 2.5 and 3.5 mm, and an inner diameter in the range between 0.5 and 2.5 mm, especially between 0.8 and 1.3 mm.

9. An orthopaedic implant, which comprises a bone screw (1) according to any one of the preceding claims or an accordingly integrally formed bone screw portion.

10. A method for producing a bone screw (1) according to any one of claims 1 to 8 or an orthopaedic implant according to claim 9, comprising the steps of:
- providing a tube or a main body having a tube portion from a magnesium alloy,
- shaping the self-tapping thread (5) in the outer wall of the tube or tube portion in order to produce the bone screw or the bone screw portion of the orthopaedic implant,
- forming a coating base (7) on at least one portion of the surface of the thread, and
- applying/introducing the microcapsules (7b) and/or the micro-abrasives (7a) to and/or into the coating base.

11. The method according to claim 10, wherein the shaping of the self-tapping thread (5) comprises rotary extrusion with subsequent milling or grinding, or comprises milling or thread tapping.

12. The method according to claim 10 or 11, wherein the forming of the coating base (7) comprises immersion in a coating liquid, in particular a liquid containing a polylactide and especially a PLA blend, and subsequent drying at elevated temperature.

13. The method according to any one of claims 10 to 12, wherein the application/introduction of the microcapsules (7b) comprises immersion in a liquid containing microcapsules with subsequent drying at elevated temperature.

14. The method according to any one of claims 10 to 13, wherein the application/introduction of the micro-abrasives (7a) comprises immersion in a liquid containing micro-abrasives with subsequent drying at elevated temperature.

15. The method according to any one of claims 10 to 14, wherein the application/introduction of the microcapsules (7b) and/or micro-abrasives (7a) comprises a prior etching of the surface of the coating base so as to create a micro-rough adhesive surface.

## Revendications

1. Vis à os (1) avec un filetage (5) auto-taraudeur, essentiellement constituée d'un alliage de magnésium et munie au moins dans des zones du filetage d'un revêtement de surface (7) bioactif qui contient des microcapsules (7b) avec un lubrifiant (7a) et/ou des micro-corps de polissage pouvant être détruits par la pression et la chaleur de frottement lors du vissage de la vis à os dans la substance osseuse.

2. Vis à os selon la revendication 1, dans laquelle les micro-corps de polissage (7a) du revêtement de surface (7) peuvent être détruits par la pression et la chaleur de frottement lors du vissage de la vis à os dans la substance osseuse, peuvent en particulier se transformer en particules plus petites qui provoquent un agrandissement de la surface de la vis effective.

3. Vis à os selon la revendication 1 ou 2, dans laquelle les micro-corps de polissage (7a) présentent de l'hydroxy apatite cristalline et sont notamment conçus en forme d'aiguilles.

4. Vis à os selon l'une des revendications précédentes, dans laquelle les microcapsules (7b) contiennent des protéines morphogénétiques osseuses.

5. Vis à os selon l'une des revendications précédentes, dans laquelle les microcapsules présentent une enveloppe de biopolymère, qui présentent en particulier un polylactide et plus spécialement un mélange de PLA.

6. Vis à os selon l'une des revendications précédentes, dans laquelle les microcapsules (7b) et/ou les micro-corps de polissage (7b), ces derniers dans leur extension la plus longue, présentent une taille dans la plage entre 0,2 et 20 µm, notamment entre 1 et 10 µm.

7. Vis à os selon l'une des revendications précédentes, dans laquelle le filetage (5) auto-taraudeur présente un profil de filetage fin en dents de scie.

8. Vis à os selon l'une des revendications précédentes, laquelle présente un corps de base en forme de canule, notamment avec un diamètre extérieur dans la plage entre 1,5 et 5 mm, plus spécialement entre 2,5 et 3,5 mm, et un diamètre intérieur dans la plage entre 0,5 et 2,5 mm, plus spécialement entre 0,8 et 1,3 mm.

9. Implant orthopédique qui présente une vis à os (1) selon l'une des revendications précédentes ou un tronçon de vis à os surmoulé correspondant.

10. Procédé de fabrication d'une vis à os (1) selon l'une des revendications 1 à 8 ou d'un implant orthopédique selon la revendication 9, avec les étapes :
- de mise à disposition d'un tube ou d'un corps de base présentant une section de tube constitué d'un alliage de magnésium,
- d'incorporation d'un filetage (5) auto-taraudeur dans la paroi extérieure du tube ou de la section de tube pour la fabrication de la vis à os ou de la section de vis à os de l'implant orthopédique,
- de formation d'une base de revêtement (7) sur au moins une section de la surface du filetage, et
- de mise en place de microcapsules (7b) et/ou de micro-corps de polissage (7a) sur et/ou dans la base de revêtement.

11. Procédé selon la revendication 10, dans lequel l'incorporation du filetage (5) auto-taraudeur présente une extrusion par rotation avec un fraisage ultérieur, ou un polissage, ou un fraisage, ou une réalisation d'un filetage.

12. Procédé selon la revendication 10 ou 11, dans lequel la formation de la base de revêtement (7) présente une immersion dans un liquide de revêtement, notamment un liquide contenant un polylactide et plus spécialement un mélange de PLA, et un séchage ultérieur à température élevée.

13. Procédé selon l'une des revendications 10 à 12, dans lequel la mise en place des microcapsules présente une immersion dans un liquide contenant des microcapsules avec un séchage ultérieur à température élevée.

14. Procédé selon l'une des revendications 10 à 13, dans lequel la mise en place des micro-corps de polissage (7a) présente une immersion dans un liquide contenant des micro-corps de polissage avec un séchage ultérieur à température élevée.

15. Procédé selon l'une des revendications 10 à 14, dans lequel la mise en place des microcapsules (7b) et/ou des micro-corps de polissage (7a) présente une attaque préalable de la surface de la base de revêtement pour la réalisation de surfaces d'adhésion micro rugueuses.
